# EUROPÄISCHE PATENTSCHRIFT

(11) **EP 0 467 251 B1**
(45) Veröffentlichungstag und Bekanntmachung des Hinweises auf die Patenterteilung: **03.06.1998**
(21) Anmeldenummer: 91111724.0
(22) Anmeldetag: 13.07.1991
(51) Int. Cl.: C07D 239/42, C07D 239/52

(54) **Verfahren zur Herstellung von N-Alkylsulfonylaminosulfonylharnstoffen**
Process for the preparation of N-alkylsulfonylaminosulfonyl ureas
Procédé de préparation de N-alkylsulfonylaminosulfonylurées

(30) Priorität: 19.07.1990 DE 4022982
(43) Veröffentlichungstag der Anmeldung: 22.01.1992
(73) Patentinhaber: Hoechst Schering AgrEvo GmbH, 13342 Berlin (DE)
(72) Erfinder: Lachhein, Stephen, Dr., W-6238 Hofheim a.Ts. (DE); Willms, Lothar, Dr., W-5416 Hillscheid (DE)

(56) Entgegenhaltungen:
- EP-A- 0 131 258
- EP-A- 0 342 456
- EP-A- 0 347 788
- EP-A- 0 353 641

## Beschreibung

Gegenstand der vorliegenden Erfindung ist ein Verfahren zur Herstellung der Verbindungen der Formel I, 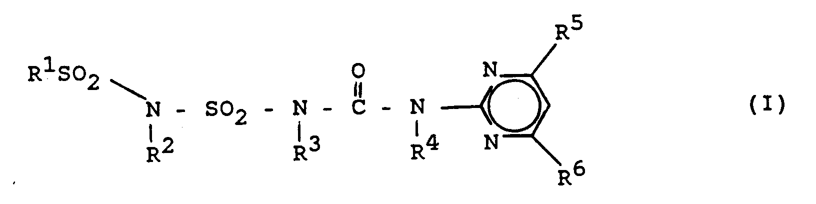 worin
- R¹: (C₁-C₆)-Alkyl, (C₂-C₆)-Alkenyl oder (C₂-C₆)-Alkinyl, wobei jeder der genannten 3 Reste unsubstituiert oder ein- oder mehrfach durch Reste aus der Gruppe Halogen, (C₁-C₄)-Alkoxy und [(C₁-C₄)-Alkoxy]-carbonyl substituiert ist,
- R²: Wasserstoff, (C₁-C₆)-Alkyl, (C₂-C₆)-Alkenyl, (C₂-C₆)-Alkinyl oder (C₃-C₆)-Cycloalkyl,
- R³, R⁴: unabhängig voneinander Wasserstoff oder (C₁-C₄)-Alkyl,
- R⁵, R⁶: unabhängig voneinander Wasserstoff, (C₁-C₄)-Alkyl oder (C₁-C₄)-Alkoxy, wobei die 2 letztgenannten Reste unsubstituiert oder ein- oder mehrfach durch Reste aus der Gruppe Halogen, Alkoxy und Alkylthio substituiert sind, Halogen, (C₁-C₄)-Alkylthio, (C₁-C₄)-Alkylamino oder (C₁-C₄)-Dialkylamino
bedeuten, sowie, falls R², R³ Wasserstoff bedeuten, deren physiologisch verträglichen Salze mit Basen.

Verbindungen der Formel I sind bekannt und werden als Pflanzenschutzmittel mit herbizider Wirkung eingesetzt; siehe z.B. EP-A 0 131 258.

Dort sind auch schon einige Verfahren zitiert oder beschrieben, nach denen Verbindungen der Formel I hergestellt werden können.

Nachteilig bei den bekannten Verfahren sind die relativ niedrigen Ausbeuten von höchstens 65-70 %. Bedingt durch diese niedrigen Ausbeuten fallen erhebliche Mengen an Verunreinigungen und Nebenprodukten an.
Diese beschriebenen Verfahren sind aus ökologischer als auch aus ökonomischer Sicht nicht in großtechnischem Maßstab durchführbar, da hierbei unvertretbar große Mengen an Nebenprodukten und Abfällen entstehen würden, die z.B. durch Verbrennung aufwendig entsorgt werden müßten. Außerdem tritt bei solch niedriger Ausbeute ein drastischer Verlust an eingesetzten Ausgangsmaterialien ein.

Es wurde nun ein neues Verfahren gefunden, nach dem die Verbindungen der Formel I in überraschend hoher Ausbeute und Reinheit herstellbar sind.

Das erfindungsgemäße Verfahren ist dadurch gekennzeichnet, daß man Verbindungen der Formel II,

R¹ - SO₂ - NR² - SO₂ - NHR³ (II)

worin
- R¹, R² und R³: wie oben definiert sind,
mit Verbindungen der Formel III, 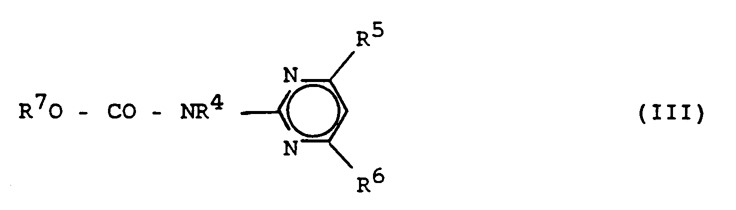 worin
- R⁴, R⁵ und R⁶: wie oben definiert sind und
- R⁷: Alkyl, Haloalkyl oder gegebenenfalls substituiertes Phenyl bedeutet,
in einem inerten organischen Lösungsmittel zu den Verbindungen der Formel I umsetzt.

In den genannten Formeln und im folgenden können Alkyl-, Alkoxy-, Haloalkyl-, Alkylamino- und Alkylthioreste sowie die entsprechenden ungesättigten und/oder substituierten Reste, wenn nicht anders angegeben, bezüglich der Kohlenstoffkette jeweils geradkettig oder verzweigt sein; Alkylreste, auch in den zusammengesetzten Bedeutungen wie Alkoxy, Haloalkyl usw., bedeuten beispielsweise Methyl, Ethyl, n- oder i-Propyl, n-, i-, t- oder 2-Butyl; Alkenyl- und Alkinylreste haben die Bedeutung der den Alkylresten entsprechenden möglichen ungesättigten Reste, wie 2-Propenyl, 2- oder 3-Butenyl, 2-Propinyl, 2- oder 3-Butinyl; gegebenenfalls substituiertes Phenyl bedeutet beispielsweise Phenyl, das unsubstituiert oder durch ein oder mehrere, vorzugsweise 1 bis 3 Reste aus der Gruppe Halogen, (C₁-C₄)-Alkyl, (C₁-C₄)-Alkoxy, (C₁-C₄)-Haloalkyl, (C₁-C₄)-Thioalkyl, (C₁-C₄-Alkoxy)-carbonyl, (C₁-C₄-Alkyl)-sulfonyl, Cyano und Nitro sustituiert ist; Halogen, auch Halo in Haloalkyl etc., bedeutet Fluor, Chlor, Brom oder Jod, vorzugsweise Fluor, Chlor oder Brom.

Unter den erfindungsgemäßen Verfahren zur Herstellung der Verbindungen der Formel I sind diejenigen bevorzugt, in denen R¹ und R² unabhängig voneinander (C₁-C₃)-Alkyl oder (C₁-C₃)-Alkenyl, insbesondere (C₁-C₂)-Alkyl, R³ und R⁴ Wasserstoff, R⁵ und R⁶ unabhängig voneinander (C₁-C₂)-Alkyl oder (C₁-C₂)-Alkoxy, insbesondere Methyl oder Methoxy, bedeuten.

R⁷ ist vorzugsweise (C₁-C₄)-Alkyl, (C₁-C₄)-Haloalkyl oder Phenyl, insbesondere Ethyl oder Phenyl.

Die Ausbeuten nach dem erfindungsgemäßen Verfahren liegen in der Regel bei mindestens 95 % d.Th. und die Reinheiten der entstehenden Sulfonylharnstoffe I sind meist größer als 98 Gew.-%.

Das erfindungsgemäße Verfahren wird in inerten organischen Lösungsmitteln durchgeführt. Beispiele für Lösungsmitteltypen sind dabei aromatische, gegebenenfalls halogenierte Kohlenwasserstoffe und aprotisch polare Lösungsmittel wie Dialkylalkanoylamide, Dialkylsulfoxide, Polyalkylenglykoldialkylether, N-alkylierte cyclische Amide und Nitrile. Bevorzugt sind Lösungsmittel wie z.B. Toluol, Xylol, Chlorbenzol, Dimethylformamid, Dimethylsulfoxid, Di-, Tri- oder Tetraethylenglykoldialkylether, insbesondere -dimethyl oder -diethylether, N-Methylpyrrolidon, Acetonitril oder auch Mischungen aus zwei oder mehreren der genannten Lösungsmittel.

In der Regel wird die Verbindung der Formel II im Verhältnis zur Verbindung der Formel III äquimolar oder in geringem Überschuß eingesetzt. Bevorzugt ist ein Molverhältnis II:III von 1:1 bis 1,2:1, insbesondere 1:1 bis 1,1:1.

Ein Vorteil des erfindungsgemäßen Verfahrens besteht darin, daß das Lösungsmittel in nahezu quantitativer Ausbeute wieder recyclisiert werden kann, weil das Produkt der Formel I als schwerlösliche Verbindung aus dem Reaktionsmedium in hoher Reinheit und Ausbeute ausfällt. Das Lösungsmittel kann anschließend z.B. durch Destillation gereinigt und dann wieder in den Produktionsprozeß eingeführt werden.

Die Reaktionstemperaturen liegen vorzugsweise bei 0°C bis zum Siedepunkt des eingesetzten Lösungsmittels, insbesondere bei Raumtemperatur (z,B. 20°C) bis 110°C.

Die für die Herstellung der erfindungsgemäßen Verbindungen der allgemeinen Formel I benötigten Ausgangsverbindungen der Formeln II und III lassen sich nach literaturbekannten Verfahren darstellen.

So werden die Verbindungen der Formel II analog üblichen Methoden (siehe z.B. "Organikum", 7. Auflage, S. 359, VEB Deutscher Verlag der Wissenschaften, Berlin 1967) durch Umsetzung der entsprechenden Sulfonamide IV mit Amidosulfonsäurechloriden V erhalten.

R¹ - SO₂ - NH - R² (IV)

Cl - SO₂ - NH - R³ (V)

Die Verbindungen der Formel III werden aus dem entsprechenden Heterocyclen VI nach laborüblichen Methoden (s. z.B. Tietze und Eicher in "Reaktionen und Synthesen" S. 92, Thieme Verlag, Stuttgart 1981) in hohen Ausbeuten durch Reaktion mit Chlorameisensäureestern VII erhalten:

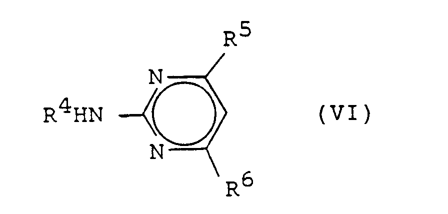

Cl - CO - OR⁷ (VII).

Die Heterocyclen der Formel VI sind entweder käuflich oder lassen sich nach geeigneten üblichen Methoden leicht herstellen; s. z.B. US 4,310,470; EP 0 024 200; US 4,299,960; M.J. Langerman, C.K. Banks, J. Am. Chem. Soc. 73, 3011 (1951).

Das Verfahren kann diskontinuierlich oder kontinuierlich durchgeführt werden.

Als besonders überraschend ist das erfindungsgemäße Verfahren deshalb anzusehen, weil die Ausgangsprodukte der Formeln II und III mehrere aktivierte elektrophile und nucleophile Zentren enthalten, die prinzipiell alle mit nucleophilen und elektrophilen Molekülteilen reagieren könnten und so durch Fragmentierungsreaktionen eine Vielzahl von Nebenprodukten liefern könnten. Die Verbindungen der Formel II besitzen insbesondere zwei Sulfonylgruppen als nahezu äquivalente Abgangsgruppen; Sulfonylgruppen sind sehr gute Abgangsgruppen (vgl. Beyer, Lehrbuch der organischen Chemie, 19. Auflage, S. 128, Hirzel Verlag Stuttgart). Erstaunlicherweise werden nach dem erfindungsgemäßen Verfahren Nebenprodukte praktisch ganz vermieden.

Somit stellt das erfindungsgemäße Verfahren einen neuen, einfachen, auch im größeren technischen Maßstab gut reproduzierbaren und hochselektiven Prozeß zur Darstellung der Verbindungen der Formel I in nahezu quantitativen Ausbeuten dar.

Das erfindungsgemäße Verfahren wird nachfolgend an einigen Beispielen erläutert. Sofern nichts anderes angegegen ist, beziehen sich Prozentangaben auf das Gewicht.

### Beispiel 1

### 1-[(N-Methylsulfonyl-N-methyl-amino)-sulfonyl]-3-(4,6-dimethoxy-2-pyrimidyl)-harnstoff

37,6 g (N-Methylsulfonyl-N-methylamino)-sulfonsäureamid werden in 500 ml Acetonitril gelöst und bei Raumtemperatur mit 55,0 g O-Phenyl-N-(4,6-Dimethoxy-2-pyrimidyl)-carbamat tropfenweise versetzt und für 18 Stunden bei Raumtemperatur gerührt. Nach Abdestillieren von ca. 400 ml Acetonitril wird mit 200 ml Wasser bei 0°C versetzt und mit 2n HCl auf pH 2-3 gestellt. Das ausgefallene Produkt wird abgesaugt und mit Wasser gewaschen. Es werden 72,6 g des gewünschten Produkts mit einer Reinheit von 98,4 % erhalten, was einer Ausbeute von 96,8 % d.Th. entspricht. Der Schmelzpunkt des Produkts ist 185-186°C.

### Beispiel 2

### 1-[(N-Methylsulfonyl-N-methyl-amino)-sulfonyl]-3-(4-methoxy-6-methyl-2-pyrimidyl)-harnstoff

37,6 g (N-Methylsulfonyl-N-methylamino)-sulfonsäureamid werden in 500 ml Chlorbenzol gelöst und die Lösung bei Raumtemperatur mit 51,8 g O-Phenyl-N-(4-Methoxy-6-methyl-2-pyrimidyl)-carbamat tropfenweise versetzt und für 5 Stunden bei 100°C gerührt. Nach Abkühlen auf 0°C wird der Niederschlag abfiltriert und nach Waschen mit 100 ml Chlorbenzol erhält man 68,8 g des gewünschten Produkts mit einer Reinheit von 98,7 %. Dies entspricht einer Ausbeute von 96,4 % d.Th. Der Schmelzpunkt des Produkts ist 118-120°C.

### Beispiel 3

### 1-[(N-Ethylsulfonyl-N-ethyl-amino)-sulfonyl]-3-(4,6-diethoxy-2-pyrimidyl)-harnstoff

40,4 g (N-Ethylsulfonyl-N-methylamino)-sulfonsäureamid werden in 500 ml Toluol gelöst und bei 100°C tropfenweise mit 51,0 g O-Ethyl-N-(4,6-Dimethoxy-2-pyrimidyl)-carbamat unter Rühren versetzt. Nach 3 Stunden Reaktionszeit bei 100°C wird auf 0°C abgekühlt und vom Niederschlag abfiltriert. Nach Waschen mit 100 ml Toluol erhält man 83,0 g des gewünschten Produkts mit einer Reinheit von 98,0 %, was einer Ausbeute von 95,7 % d.Th. entspricht. Der Schmelzpunkt des Produkts ist 175-176°C.

Analog den Beispielen 1 bis 3 werden die in nachfolgender Tabelle 1 aufgeführten Verbindungen der Formel I synthetisiert.

**Tabelle 1**

| Bsp. Nr. | R¹ | R² | R³ | R⁴ | R⁵ | R⁶ | Smp. (°C) |
|---|---|---|---|---|---|---|---|
| 4 | CH₃ | CH₃ | H | H | CH₃ | CH₃ | 150-151 |
| 5 | CH₃ | C₃H₇ | H | H | CH₃ | CH₃ | 149-151 |
| 6 | CH₃ | C₃H₇ | H | H | OCH₃ | CH₃ | 141-143 |
| 7 | CH₃ | CH₂=CHCH₂ | H | H | CH₃ | CH₃ | 139-141 |
| 8 | CH₃ | CH₂=CHCH₂ | H | H | OCH₃ | CH₃ | 159-161 |
| 9 | CH₂Cl | CH₃ | H | H | CH₃ | CH₃ | 146-148 |
| 10 | CH₃ | C₃H₇ | H | H | OCH₃ | OCH₃ | 156-157 |
| 11 | CH₃ | CH(CH₃)₂ | H | H | OCH₃ | OCH₃ | 121-123 |
| 12 | CH₃ | CH(CH₃)₂ | H | H | Cl | OCH₃ | 153-155 |
| 13 | C₂H₅ | C₂H₅ | H | H | OCH₃ | OCH₃ | |
| 14 | CH₃ | CH₃ | CH₃ | H | OCH₃ | OC₂H₅ | |
| 15 | CH₃ | CH₃ | H | CH₃ | OCH₃ | OCH₃ | |
| 16 | CH₃ | C₂H₅ | CH₃ | CH₃ | C₂H₅ | OCH₃ | |
| 17 | C₃H₇ | CH₃ | H | H | CH₃ | CH₃ | |
| 18 | C₄H₉ | CH₃ | H | H | OCH₃ | OCH₃ | |
| 19 | CH₃ | cyclo-C₆H₁₁ | H | H | OCH₃ | OCH₃ | |
| 20 | CH₃ | CH₂-C≡CH | CH₃ | H | CH₃ | OCF₂H | |
| 21 | CH₃ | CH₂-CO₂CH₃ | H | H | CH₃ | CH₃ | |

## Patentansprüche

1. Verfahren zur Herstellung von Verbindungen der Formel I 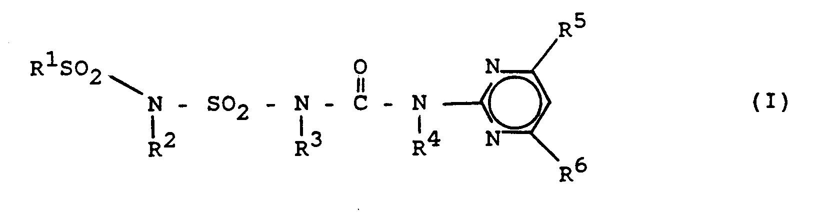 worin
R¹ (C₁-C₆)-Alkyl, (C₂-C₆)-Alkenyl oder (C₂-C₆)-Alkinyl, wobei jeder der genannten 3 Reste unsubstituiert oder ein- oder mehrfach durch Halogen, (C₁-C₄)-Alkoxy und [(C₁-C₄)-Alkoxy]-carbonyl substituiert ist,
R² Wasserstoff, (C₁-C₆)-Alkyl, (C₂-C₆)-Alkenyl, (C₂-C₆)-Alkinyl oder (C₃-C₆)-Cycloalkyl,
R³, R⁴ unabhängig voneinander Wasserstoff oder (C₁-C₄)-Alkyl,
R⁵, R⁶ unabhängig voneinander Wasserstoff, (C₁-C₄)-Alkyl oder (C₁-C₄)-Alkoxy, wobei die 2 letztgenannten Reste unsubstituiert oder ein- oder mehrfach durch Reste aus der Gruppe Halogen, Alkoxy und Alkylthio substituiert sind, Halogen, (C₁-C₄)-Alkylthio, (C₁-C₄)-Alkylamino oder (C₁-C₄)-Dialkylamino
bedeuten, sowie, falls R², R³ Wasserstoff bedeuten, deren physiologisch verträglichen Salzen mit Basen; dadurch gekennzeichnet, daß man Verbindungen der Formel II,
R¹ - SO₂ - NR² - SO₂ - NHR³ (II)
worin
R¹, R² und R³ wie oben definiert sind, mit Verbindungen der Formel III, 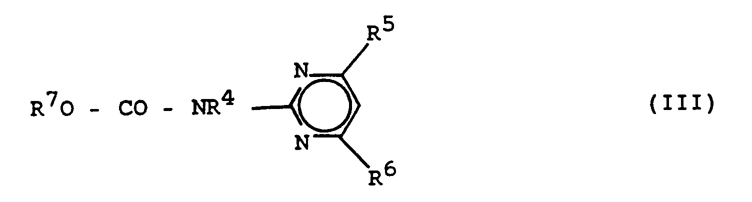 worin
R⁴, R⁵ und R⁶ wie oben definiert sind und
R⁷ Alkyl, Haloalkyl oder gegebenenfalls substituiertes Phenyl bedeutet,
in einem inerten organischen Lösungsmittel zu den Verbindungen der Formel I umsetzt.

2. Verfahren nach Anspruch 1, dadurch gekennzeichnet, daß R¹ und R² unabhängig voneinander (C₁-C₃)-Alkyl oder (C₁-C₃)-Alkenyl, R³ und R⁴ Wasserstoff, R⁵, R⁶ unabhängig voneinander (C₁-C₂)-Alkyl oder (C₁-C₂)-Alkoxy bedeuten.

3. Verfahren nach Anspruch 1 oder 2, dadurch gekennzeichnet, daß R¹ (C₁-C₂)-Alkyl, R² (C₁-C₃)-Alkyl oder (C₁-C₃)-Alkenyl, R³ und R⁴ Wasserstoff, R⁵ Methyl oder Methoxy und R⁶ Methyl oder Methoxy bedeuten.

4. Verfahren nach einem oder mehreren der Ansprüche 1 bis 3, dadurch gekennzeichnet, daß R⁷ (C₁-C₄)-Alkyl, (C₁-C₄)-Haloalkyl oder Phenyl ist.

5. Verfahren nach einem oder mehreren der Ansprüche 1 bis 4, dadurch gekennzeichnet, daß man als inerte organische Lösungsmittel aromatische Kohlenwasserstoffe, halogenierte aromatische Kohlenwasserstoffe oder aprotisch polare Lösungsmittel oder Mischungen der genannten Lösungsmittel einsetzt.

6. Verfahren nach einem oder mehreren der Ansprüche 1 bis 5, dadurch gekennzeichnet, daß man es bei Reaktionstemperaturen von 0°C bis zum Siedepunkt des eingesetzten Lösungsmittels durchführt.

7. Verfahren nach Anspruch 6, dadurch gekennzeichnet, daß die Reaktionstemperatur Raumtemperatur bis 110°C ist.

8. Verfahren nach einem oder mehreren der Ansprüche 1 bis 7, dadurch gekennzeichnet, daß die Verbindungen II und III im Molverhältnis von 1:1 bis 1,2:1 umgesetzt werden.

9. Verfahren nach Anspruch 8, dadurch gekennzeichnet, daß das Molverhältnis 1:1 bis 1,1:1 ist.

10. Verfahren nach einem oder mehreren der Ansprüche 1 bis 9, dadurch gekennzeichnet, daß das Verfahren diskontinuierlich oder kontinuierlich durchgeführt wird.

## Claims

1. A process for preparing compounds of the formula I 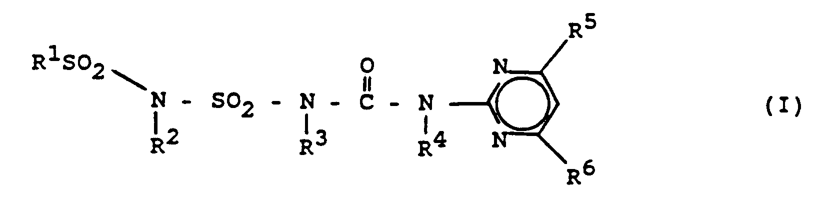 in which
R¹ is (C₁-C₆)-alkyl, (C₂-C₆)-alkenyl or (C₂-C₆)-alkinyl, where each of the 3 radicals mentioned is unsubstituted or mono- or polysubstituted by halogen, (C₁-C₄)-alkoxy and [(C₁-C₄)-alkoxy]-carbonyl,
R² is hydrogen, (C₁-C₆)-alkyl, (C₂-C₆)-alkenyl, (C₂-C₆)-alkinyl or (C₃-C₆)-cycloalkyl,
R³, R⁴ independently of one another are hydrogen or (C₁-C₄)-alkyl,
R⁵, R⁶ independently of one another are hydrogen, (C₁-C₄)-alkyl or (C₁-C₄)-alkoxy, where the 2 last mentioned radicals are unsubstituted or mono- or polysubstituted by radicals from the group consisting of halogen, alkoxy and alkylthio, are halogen, (C₁-C₄)-alkylthio, (C₁-C₄)-alkylamino or (C₁-C₄)-dialkylamino
and if R², R³ are hydrogen, their physiologically acceptable salts with bases, which comprises reacting compounds of the formula II
R¹ - SO₂ - NR² - SO₂ - NHR³ (II),
in which
R¹, R² and R³ are as defined above, with compounds of the formula III 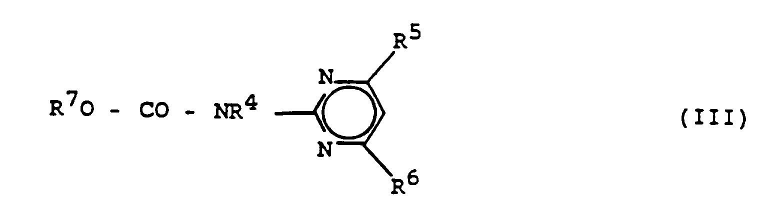 in which
R⁴, R⁵ and R⁶ are as defined above and
R⁷ is alkyl, haloalkyl or unsubstituted or substituted phenyl,
in an inert organic solvent to give compounds of the formula I.

2. The process as claimed in claim 1, wherein R¹ and R² independently of one another are (C₁-C₃)-alkyl or (C₁-C₃)-alkenyl, R³ and R⁴ are hydrogen, R⁵ and R⁶ independently of one another are (C₁-C₂)-alkyl or (C₁-C₂)-alkoxy.

3. The process as claimed in claim 1 or 2, wherein R¹ is (C₁-C₂)-alkyl, R² is (C₁-C₃)-alkyl or (C₁-C₃)-alkenyl, R³ and R⁴ are hydrogen, R⁵ is methyl or methoxy and R⁶ is methyl or methoxy.

4. The process as claimed in one or more of claims 1 to 3, wherein R⁷ is (C₁-C₄)-alkyl, (C₁-C₄)-haloalkyl or phenyl.

5. The process as claimed in one or more of claims 1 to 4, wherein the inert organic solvents used are aromatic hydrocarbons, halogenated aromatic hydrocarbons or aprotic polar solvents or mixtures of these.

6. The process as claimed in one or more of claims 1 to 5, wherein the process is carried out at reaction temperatures of from 0°C to the boiling point of the solvent employed.

7. The process as claimed in claim 6, wherein the reaction temperature is from room temperature to 110°C.

8. The process as claimed in one or more of claims 1 to 7, wherein the compounds II and III are reacted in a molar ratio of 1:1 to 1.2:1.

9. The process as claimed in claim 8, wherein the molar ratio is 1:1 to 1.1:1.

10. The process as claimed in one or more of claims 1 to 9, wherein the process is carried out batchwise or continuously.

## Revendications

1. Procédé de préparation de composés de formule I : 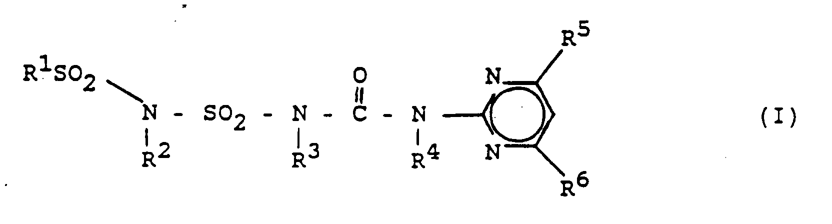 dans laquelle
R¹ représente un groupe alkyle en C₁-C₆, alcényle en C₂-C₆ ou alcynyle en C₂-C₆, chacun des 3 radicaux cités étant non substitué ou une ou plusieurs fois substitué par un atome d'halogène, un groupe alkoxy en C₁-C₄ et (alkoxy en C₁-C₄)-carbonyle,
R² représente un atome d'hydrogène, un groupe alkyle en C₁-C₆, alcényle en C₂-C₆, aloynyle en C₂-C₆ ou cycloalkyle en C₃-C₆,
R³, R⁴ représentent indépendamment l'un de l'autre un atome d'hydrogène ou un groupe alkyle en C₁-C₄,
R⁵, R⁶ représentent indépendamment l'un de l'autre un atome d'hydrogène, un groupe alkyle en C₁-C₄ ou alkoxy en C₁-C₄, les 2 derniers radicaux cités étant non substitués ou une ou plusieurs fois substitués par des résidus choisis parmi un atome d'halogène, un groupe alkoxy et alkylthio, ou représentent un atome d'halogène, un groupe alkylthio en C₁-C₄, alkylamino en C₁-C₄ ou dialkylamino en C₁-C₄,
ainsi que, dans le cas où R², R³ représentent des atomes d'hydrogène, de leurs sels physiologiquement acceptables avec des bases, caractérisé en ce que, l'on fait réagir des composés de formule II,
R¹ - SO₂ - NR² - SO₂ - NHR³ (II)
dans laquelle
R¹, R² et R³ sont définis comme ci-dessus, avec des composés de formule III, 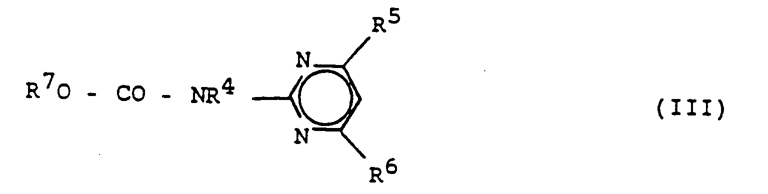 dans laquelle
R⁴, R⁵ et R⁶ sont définis comme ci-dessus et
R⁷ représente un groupe alkyle, haloalkyle, ou phényle éventuellement substitué,
dans un solvant organique inerte pour donner les composés de formule I.

2. Procédé selon la revendication 1, caractérisé en ce que, R¹ et R² représentent, indépendamment l'un de l'autre, un groupe alkyle en C₁-C₃ ou alcényle en C₁-C₃, R³ et R⁴ un atome d'hydrogène, R⁵ et R⁶ indépendamment l'un de l'autre un groupe alkyle en C₁-C₂ ou alkoxy en C₁-C₂.

3. Procédé selon la revendication 1 ou 2, caractérisé en ce que, R¹ représente un groupe alkyle en C₁-C₂, R² un groupe alkyle en C₁-C₃ ou alcényle en C₁-C₃, R³ et R⁴ un atome d'hydrogène, R⁵ un groupe méthyle ou méthoxy et R⁶ un groupe méthyle ou méthoxy.

4. Procédé selon une ou plusieurs des revendications 1 à 3, caractérisé en ce que, R⁷ est un groupe alkyle en C₁-C₄, haloalkyle en C₁-C₄ ou phényle.

5. Procédé selon une ou plusieurs des revendications 1 à 4, caractérisé en ce que l'on utilise comme solvant organique inerte des hydrocarbures aromatiques, des hydrocarbures aromatiques halogénés ou des solvants polaires aprotiques ou des mélanges des solvants cités.

6. Procédé selon une ou plusieurs des revendications 1 à 5, caractérisé en ce qu'on le réalise à des températures de réaction de 0°C à la température d'ébullition du solvant utilisé.

7. Procédé selon la revendication 6, caractérisé en ce que la température de réaction est entre la température ambiante et 110°C.

8. Procédé selon une ou plusieurs des revendications 1 à 7, caractérisé en ce que l'on fait réagir les composés II et III dans le rapport molaire de 1:1 à 1,2:1.

9. Procédé selon la revendication 8, caractérisé en ce que le rapport molaire est de 1:1 à 1,1:1.

10. Procédé selon une ou plusieurs des revendications 1 à 9, caractérisé en ce que l'on réalise le procédé en discontinu ou en continu.
